# EUROPÄISCHE PATENTSCHRIFT

(11) **EP 0 742 716 B1**
(45) Veröffentlichungstag und Bekanntmachung des Hinweises auf die Patenterteilung: **12.03.2003**
(21) Anmeldenummer: 95905121.0
(22) Anmeldetag: 05.01.1995
(51) Int. Cl.: A61K 31/485, A61K 9/70

(54) **PHARMAZEUTISCHE ZUSAMMENSETZUNG ZUR SYSTEMISCHEN TRANSDERMALEN VERABREICHUNG MIT DEM WIRKSTOFF MORPHIN-6-GLUCURONID**
PHARMACEUTICAL COMPOSITION FOR SYSTEMIC TRANSDERMAL ADMINISTRATION CONTAINING THE ACTIVE AGENT MORPHINE-6-GLUCURONIDE
COMPOSITION PHARMACEUTIQUE POUR ADMINISTRATION TRANSDERMIQUE SYSTEMIQUE CONTENANT DU MORPHINE-6-GLUCORONIDE COMME PRINCIPE ACTIF

(30) Priorität: 07.02.1994 DE 4403709
(43) Veröffentlichungstag der Anmeldung: 20.11.1996
(73) Patentinhaber: LTS Lohmann Therapie-Systeme AG, 56626 Andernach (DE)
(72) Erfinder: HILLE, Thomas, D-56564 Neuwied (DE); OTTO, Karlheinz, D-83043 Bad Aibling (DE)
(74) Vertreter: Flaccus, Rolf-Dieter, Dr.
(86) Internationale Anmeldenummer: EP9500031
(87) Internationale Veröffentlichungsnummer: WO95020966

(56) Entgegenhaltungen:
- WO-A-88/01497
- WO-A-92/08459
- WO-A-93/15737
- WO-A-95/05831
- J. PHARMACOL. EXP. THER., Bd.262, Nr.1, 1992 Seiten 25 - 31 B. FRANCES 'Further evidence that morphine-6- -glucuronide is a more potent opioid agonist than morphine.'

## Beschreibung

Die Erfindung betrifft die systemische transdermale Gabe von Morphin-6-glucuronid.

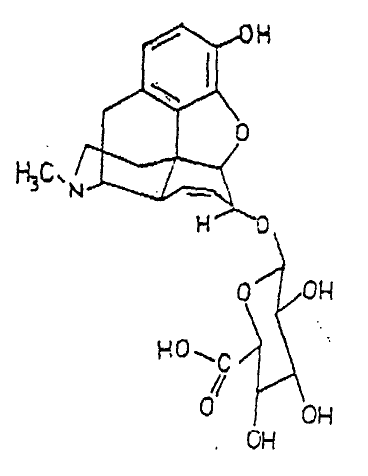

Morphin-6-glucuronid (Glucuronsäure-6-(7,8-Didehydro-4,5 epoxy-17-methylmorphinan-3,6 diolyl) ester) ist in der Literatur als aktiver Metabolit von Morphin beschrieben, der wirksamer ist als Morphin selbst (Oguri, K., J. pharmal. Soc. Japan [Yakugaku Zasshi] 100, 117 (1980)). Die Synthese von Morphin-6-glucuronid sowie der pharmazeutisch verwendbaren Säureadditionssalze ist in WO-9305057 und WO-9303051 beschrieben.

Zur Behandlung von chronischen Schmerzen, z.B. bei Tumorpatienten, ist Morphin der Arzneistoff der Wahl, wobei die Verabreichung der relativ hohen Tagesdosen auf Schwierigkeiten stößt. Obwohl das Morphin-6-glucuronid eine höhere Wirksamkeit besitzt, steckt seine medizinische Anwendung noch in den Anfängen. Somit ist die Aufgabe der Erfindung, eine Möglichkeit zu finden, das Morphin-6-glucuronid in einfacher Weise und in der erforderlichen Dosis zur Applikation zu bringen.

Diese Aufgabe wird überraschenderweise dadurch gelöst, daß das Morphin-6-glucuronid oder seine Salze transdermal verabreicht werden. Diese Lösung ist umso erstaunlicher, da entgegen allen Erwartungen Morphin-6-glucuronid leichter durch Haut diffundiert als das kleinere Morphin.

Die erhöhte Hydrophilie und Polarität einer glucuronierten Verbindung gegenüber der Stammverbindung und die damit verbundene geringere Hautgängigkeit ließ dieses Ergebnis nicht erwarten.

WO-A-88/01497 beschreibt transdermale Darreichungsformen für Morphinan-Analgetika, wobei diese Wirkstoffe in Form der freien Basen oder als pharmazeutisch akzeptable Säureadditionssalze in den Zubereitungen vorliegen können. Konjugate bzw. Metabolite von Morphin-Analgetika werden nicht in Betracht gezogen.
WO-A-93/15737 betrifft Zubereitungen für die nasale Verabreichung von polaren Metaboliten von Opiat-Analgetika, beispielsweise von Morphin-6-glucuronid. Eine transdermale Verabreichung ist nicht vorgesehen.

Da Morphin-6-glucuronid ein aktiver Metabolit des Morphins ist, ist es toxikologisch wie dieses zu beurteilen. Hier liegt der Vorteil von Morphin-6-glucuronid im Vergleich zu anderen Opioiden, wie z.B. Fentanyl und seinen Derivaten.

Unter dem Begriff "pharmazeutische Zusammensetzung zur transdermalen Verabreichung" wird jede konventionelle flüssige, halbflüssige oder feste Zubereitung, z.B. Lösungen, Salben, Pasten, Gele, Tinkturen und andere, oder polymerhaltige Matrices verstanden.

Unter einem transdermalen therapeutischen System (TTS) soll nach Zaffaroni "eine arzneistoffenthaltende Vorrichtung bzw. eine Darreichungsform, die einen Arzneistoff oder mehrere in vorausbestimmter Rate kontinuierlich über einen festgesetzten Zeitraum an einen festgelegten Anwendungsort abgibt" (zitiert nach: Heilmann, Klaus: Therapeutische Systeme - Konzept und Realisation programmierter Arzneiverabreichung, 4. Auflage, Ferdinand Enke Verlag, Stuttgart 1984, S. 26), verstanden werden, wobei im vorliegenden Fall der Anwendungsort auf der Haut liegt.

Der Aufbau von transdermalen Systemen ist dem Fachmann bekannt. Schutzrechte, in denen der grundsätzliche Aufbau beschrieben wird, sind beispielsweise DE 33 15 272, DE 38 43 239 und US 3,598,122.

Wird ein transdermales therapeutisches System auf die Haut eines Patienten appliziert, so soll der Arzneistoff abgegeben werden, um beim Patienten topisch oder systemisch wirksam zu werden. Arzneiformen dieser Art werden bereits therapeutisch genutzt. Das besonders bevorzugte transdermale System in Pflasterform besteht aus einer wirkstoffundurchlässigen Rückschicht, einer haftklebenden Reservoirschicht und gegebenenfalls einer wieder ablösbaren Schutzschicht.

Dabei kann die wirkstoffundurchlässige Rückschicht aus flexiblem oder nicht flexiblem Material bestehen. Substanzen, die zu ihrer Herstellung verwendet werden können, sind Polymerfolien oder Metallfolien, wie Aluminiumfolie, die allein oder mit einem polymeren Substrat beschichtet, angewandt werden. Es können auch textile Flächengebilde verwendet werden, wenn die Bestandteile des Reservoirs aufgrund ihrer physikalischen Beschaffenheit durch sie nicht hindurchtreten können. Bei einer bevorzugten Ausführungsform ist die Rückschicht ein Verbundstoff aus einem mit Aluminium bedampften Folie.

Die Reservoirschicht enthält eine Polymermatrix und den Wirkstoff, wobei die Polymermatrix die Eigenschaft besitzt, den Zusammenhalt des Systems zu gewährleisten. Sie besteht aus einem Grundpolymer und gegebenenfalls den üblichen Zusätzen. Die Auswahl des Grundpolymers richtet sich nach den chemischen und physikalischen Eigenschaften des Wirkstoffs. Beispielhafte Polymere sind Kautschuk, kautschukähnliche, synthetische Homo-, Co- oder Blockpolymere, Polyacrylsäureester und deren Copolymere. Grundsätzlich kommen alle Polymere in Frage, die bei der Herstellung von Haftklebern eingesetzt werden, physiologisch unbedenklich sind und Morphin-6-glucuronid nicht zersetzen. Besonders bevorzugt sind solche, die als Blockcopolymeren auf Basis von Styrol und 1,3-Dienen, Polyisobutylenen oder Polymeren aus Acrylatund/oder Methacrylat bestehen. Von den Blockpolymeren auf Basis von Styrol und 1,3-Dienen werden ganz besonders lineare Styrol-Isopren-Blockcopolymere eingesetzt.

Als Polymere auf Acrylat-Basis werden Acrylatcopolymere aus 2-Ethylhexylacrylat, Vinylacetat und Acrylsäure mit bzw. ohne Chelatester bevorzugt. Als Methylacrylate werden Copolymere auf Basis von Dimethylaminoethylmethacrylaten und neutralen Methacrylsäureestern bevorzugt.

Die Art der möglichen Zusätze hängt vom eingesetzten Polymer und dem Wirkstoff ab: Nach ihrer Funktion lassen sie sich einteilen in Weichmacher, Klebrigmacher, Stabilisatoren, Trägerstoffe, diffusions- und penetrationsregulierende Zusätze oder Füllstoffe. Die hierfür in Frage kommenden physiologisch unbedenklichen Substanzen sind dem Fachmann bekannt. Die Reservoirschicht besitzt eine solche Eigenklebrigkeit, daß ein dauernder Kontakt zur Haut sichergestellt ist. Besonders bevorzugte Klebrigmacher sind Ester von Kolophonium. Als Ester von hydriertem Kolophonium werden vorzugsweise insbesondere dessen Methyl- und Glycerinester verwendet.

Beispiele für geeignete Weichmacher sind Diester von Dicarbonsäure, z.B. Di-n-butyladipat, sowie Triglyceride, insbesondere mittelkettige Triglyceride der Capryl/Caprinsäure des Kokosöls sind zu nennen. Weiter Beispiele für einen geeigneten Weichmacher sind Isopropylmyristat, Dioctylcyclohexan u.a.

Die ablösbare Schutzschicht, die mit der Reservoirschicht in Berührung steht und vor der Anwendung entfernt wird, besteht beispielsweise aus denselben Materialien, wie sie zur Herstellung der Rückschicht benutzt werden, vorausgesetzt, daß sie ablösbar gemacht werden, wie z.B. durch eine Siliconbehandlung. Andere ablösbare Schutzschichten sind z.B. Polytetrafluorethylen, behandeltes Papier, Cellophan, Polyvinylchlorid u.ä. Wird das erfindungsgemäße Laminat vor Aufbringen der Schutzschicht in therapiegerechte Formate (Pflaster) aufgeteilt, so können die dann aufzubringenden Schutzschichtformate ein überstehendes Ende aufweisen, mit dessen Hilfe sie leichter von dem Pflaster abgezogen werden können.

Das erfindungsgemäße transdermale System wird hergestellt, indem der Wirkstoff zusammen mit den Bestandteilen der haftklebenden Reservoirschicht gegebenenfalls in Lösung homogen vermischt und auf die wirkstoffundurchlässige Rückschicht aufgestrichen wird, worauf gegebenenfalls das Lösemittel oder die Lösemittel entfernt wird/werden. Anschließend wird die Klebeschicht mit einer entsprechenden Schutzschicht versehen.

Ein bevorzugtes Säureadditionssalz ist das Hydrochlorid. Morphin-6-glucuronid und seine Salze werden demnach zur Herstellung eines Mittels zur systemischen transdermalen Verabreichung zur Behandlung von starken Schmerzen verwendet.

### Die Erfindung wird durch folgendes Beispiel erläutert:

### Beispiel:

20 g n-Heptan und 80 g Methylethylketon werden gemischt. In 90 g dieser Mischung löst man 7,2 g Morphin-6-glucuronid. Nach vollständiger Auflösung des Wirkstoffes gibt man portionsweise 40 g eines linearen Styrol-Isopren-Styrol-Blockpolymers, sowie 5,6 g Triglyceride der Capryl/Caprinsäuren des Kokosöls ("Mittelkettige Triglyceride", DAB 10) zu. Unter Lichtausschluß rührt man bei Raumtemperatur 8 Stunden lang bis zur vollständigen Auflösung und streicht die erhaltene Lösung mit einer 250 µm Rakel auf eine aluminisierte und silikonisierte Polyethylen-Folie.

Nachdem das Lösemittel durch 25-minütiges Trocknen bei 50°C entfernt wurde, deckt man den Klebefilm mit einer Polyester-Folie 15 µm ab. Mit geeigneten Schneidewerkzeugen stanzt man eine Fläche von 16 cm² aus und entfernt die Ränder durch Abgittern.

Die Stabilität des Wirkstoffes im System wurde durch Gehaltsbestimmung direkt nach der Fertigung bzw. nach dreimonatiger Lagerung aufgezeigt. Dabei konnten weder die aus der Literatur bekannten Abbauprodukte Morphinbase und Glucuronsäure noch andere bisher nicht beschriebene detektiert werden. Dazu wurde folgende Methode angewandt:

### Probenvorbereitung:

Ein Pflaster mit Abdeckfolie wird mittels Schere geviertelt, die Abdeckfolie entfernt und zusammen mit den Pflasterteilen in einem verschließbaren, lichtgeschützten Glasgefäß mit 50,0 ml Tetrahydrofuren (p.a.) mindestens 2 Stunden geschüttelt. 10 min ultrabeschallt und anschließend zentrifugiert. Verdünnung für HPLC mit Methanol und nochmalige Zentrifugation.

Anschließend wird der Gehalt des Morphin-6-glucuronids im Zentrifugat per HPLC bestimmt.

Für die Stabilität des Wirkstoffes ist entscheidend, daß die eingesetzten Polymere, Harze und Weichmacher weder freie Hydroxylgruppen noch Polyethoxygruppen enthalten, da der Anteil an Wirkstoff, der gelöst vorliegt, sonst der Hydrolyse unterliegen würde. Daher wurden Harze und Weichmacher der Verbindungsklasse Ester gewählt.

Für die Stabilität des Wirkstoffes ist auch die Wahl des Lösemittels bzw. des Lösemittelgemisches entscheidend, wenn dieses vor der Trocknung über Stunden auf den Wirkstoff einwirkt. Der Anteil des zur Unterdrückung der Blasenbildung gegebenenfalls erforderlichen höher siedenden Lösemittels muß gering sein. Dies wird in dem vorliegenden Beispiel dadurch erreicht, daß eine Mischung aus Butanon und n-Heptan gewählt wurde, die ein azeotropes Gemisch (Verhältnis Butanon: n-Heptan 70 : 30, Siedepunkt 77°C; Siedepunkt Butanon: 79,6 °C; Siedepunkt n-Heptan: 98,5 °C) bilden. Dadurch kann trotz schonender Trocknung eine maximale Restfeuchte kleiner als 0,4% erreicht werden.

Mit dem Rezepturbeispiel wurde in-vitro ein Permeationsexperiment durchgeführt. Hierzu wurde ein dem Fachmann bekannter in-vitro-Standard-Test benutzt. Als Hautmodell diente excidierte Mäusehaut, als Akzeptormedium physiologische Kochsalzlösung und als Apparatur die Diffusionszelle nach Franz (Franz, T.J., J. Invest. Dermatol. 64, 194-195, (1975)). Die Formulierung wurde auf die eine Seite von intakter Haut aufgebracht, während die andere in Kontakt mit physiologischer Kochsalzlösung steht. Die Gehaltsbestimmung im Akzeptormedium erfolgt per HPLC. Es wurde ein Flux von 2,871 mg/2,54 cm² x 24 h erzielt, der bei Morphin aus einem analogen System bei 1,886 mg/2,54 cm² x 24 h liegt.

Excidierte Mäusehaut ist dem Fachmann als Modell für invitro-Tests bekannt. Durch den hohen erzielten Wert ist davon auszugehen, daß Morphin-6-glucuronid auch in-vitro leichter durch die menschliche Haut diffundiert als Morphin, mit dem solcher Flux nicht erreicht wird.

## Patentansprüche

1. Pharmazeutische Zusammensetzung zur systemischen transdermalen Verabreichung, **dadurch gekennzeichnet, daß** sie ein transdermales therapeutisches System in Form eines Pflasters ist, und als aktives Mittel Morphin-6-glucuronid oder ein pharmazeutisch verwendbares Säureadditionssalz davon enthält.

2. Pharmazeutische Zusammensetzung nach Anspruch 1, **dadurch gekennzeichnet, daß** das aktive Mittel das Hydrochlorid ist.

3. Verwendung von Morphin-6-glucuronid oder eines seiner pharmazeutisch verwendbaren Säureadditionssalze zur Herstellung einer pharmazeutischen Zusammensetzung nach Anspruch 1.

4. Verwendung nach Anspruch 3, **dadurch gekennzeichnet, daß** die pharmazeutische Zubereitung zur Behandlung starker Schmerzen geeignet ist.

## Claims

1. A pharmaceutical composition for the systemic transdermal administration **characterized in that** it is a transdermal therapeutic system in the form of a patch and contains morphine-6-glucuronide or a pharmaceutically applicable acid addition salt thereof as an active agent.

2. The pharmaceutical composition according to claim 1 **characterized in that** the active agent is the hydrochloride.

3. The use of morphine-6-glucuronide or of one of its pharmaceutically applicable acid addition salts for the production of a pharmaceutical composition according to claim 1.

4. Use according to Claim 3 **characterized in that** the pharmaceutical preparation is suitable for the treatment of intense pain.

## Revendications

1. Composition pharmaceutique pour l'administration transdermique systémique, **caractérisée en ce qu'**un système thérapeutique transdermique se trouve sous la forme d'un emplâtre, et contient en tant que principe actif, le morphine-6-giucuronide ou un sel d'addition d'acide applicable sur le plan pharmaceutique.

2. Composition pharmaceutique selon la revendication 1, **caractérisée en ce que** le principe actif est le chlorhydrate.

3. Emploi du morphine-6-glucuronide ou un de ses sels d'addition d'acide utilisable sur le plan pharmaceutique, pour la préparation d'une composition pharmaceutique selon la revendication 1.

4. Utilisation selon la revendication 3, **caractérisée en ce que** la préparation pharmaceutique convient au traitement de douleurs intenses.
